# EUROPEAN PATENT APPLICATION

(11) **EP 1 829 567 A1**
(43) Date of publication of application: **05.09.2007**
(21) Application number: 05814677.0
(22) Date of filing: 06.12.2005
(51) Int. Cl.: A61L 33/00, A61M 25/00

(54) **MEDICAL INSTRUMENT**

(30) Priority: 21.12.2004 JP 2004370072
(71) Applicant: Shinshu University, Matsumoto-shi, Nagano 390-8621 (JP); MEFS KABUSHIKI KAISHA, Nagano-shi, Nagano, 3800921 (JP)
(72) Inventor: ENDO, Morinobu, 3820028 (JP); KOYAMA, Shozo, 3900221 (JP); ICHINOSE, Masao, c/o MEFS KABUSHIKI KAISHA, Ooaza kurita, Nagano-shi, Nagano 3800921 (JP)
(74) Representative: Calderbank, Thomas Roger
(86) International application number: PCT/JP2005/022347
(87) International publication number: WO 2006/067959

(57) **Abstract**

The present invention provides a medical instrument with use of a composite material, which has antithrombotic characteristics and biocompatibility, has a favorable strength per se and is excellent in handling properties. A part or the whole of a section of the medical instrument, which is brought into contact with blood, is made of or coated with the composite material, which has antithrombotic characteristics and in which carbon nanotubes are blended with matrix resin.

## Description

### FIELD OF TECHNOLOGY

The present invention relates to a medical instrument, more precisely relates to a medical instrument with use of a composite material, which has antithrombotic characteristics.

### BACKGROUND TECHNOLOGY

Forming thrombus on a surface of a medical instrument used in and outside of a living body, e.g., catheter, is an unavoidable phenomenon. A mechanism of thrombus formation is overly complicated, and all components of blood participate in the thrombus formation on a surface of a biological material. To solve the problem, some researchers are studying the mechanism of thrombus formation, and some other researchers are studying new materials for medical instruments, which are capable of restraining thrombus formation.

Japanese Patent Kokai Gazette No. 2001-29447 (Patent Document 1) discloses a technical idea of coating a surface of a medical instrument with a diamond-like carbon film including specific fluorine.

Japanese Patent Kohyo Gazette No. 11-502734 (Patent Document 2) discloses a technical idea of coating a surface of a medical instrument with hydrophilic polymer including an antithrombotic drug.

Japanese Patent Kohyo Gazette No. 2004-512397 (Patent Document 3) discloses a technical idea of coating a surface of a medical instrument with hydrophobic multicomponant heparin conjugates.

Japanese Patent Kokai Gazette No. 7-16292 (Patent Document 4) discloses a material of a special antithrombotic medical instrument made of graft copolymer.

Further, Japanese Patent Kokai Gazette No. 6-277293 (Patent Document 5) discloses substances constituted by polyether block amid copolymer, polyurethane and an X-ray contrast agent.
Patent Document 1: Japanese Patent Kokai Gazette No. 2001-29447
Patent Document 2: Japanese Patent Kohyo Gazette No. 11-502734
Patent Document 3: Japanese Patent Kohyo Gazette No. 2004-512397
Patent Document 4: Japanese Patent Kokai Gazette No. 7-16292
Patent Document 5: Japanese Patent Kokai Gazette No. 6-277293

### DISCLOSURE OF THE INVENTION

The above described new materials are made of special substances and produced by a complicated manner, further they are expansive and hard to handle.

The present invention provides a medical instrument with use of a composite material, which has antithrombotic characteristics and biocompatibility, has a favorable strength per se and is excellent in handling properties.

The medical instrument of the present invention has a section, a part of or the whole of which is brought into contact with blood, and the section is made of or coated with a composite material, which has antithrombotic characteristics and in which carbon nanotubes are blended with matrix resin.

In the medical instrument, 1-30 wt% of carbon nanotubes may be blended with the matrix resin in the composite material.

Preferably, the carbon nanotubes are graphitized.

In the medical instrument, the carbon nanotubes may be produced by a vapor growth method, in which a hydrogen carbonate gas is vapor-thermal-decomposed in the presence of a ferrous catalyst.

Preferably, content of iron in the carbon nanotubes is 500 ppm or less.

The medical instrument may be an implantable instrument, such as an artificial heart, a tube for transporting a drug and a stent.

Further, medical instrument may be an external instrument used outside of a living body, such as an instrument for blood examination and a surgical instrument.

### EFFECTS OF THE INVENTION

In the composite material in which carbon nanotubes (CNT) are blended with matrix resin, the CNTs act as cores for crystallizing the matrix resin and accelerate the crystallization of the matrix resin, so that a surface of the matrix resin is made dense and smooth; reactivity with blood is lowered, and excellent antithrombotic characteristics can be gained. Therefore, the medical instrument having the section, a part of or the whole of which is brought into contact with blood and which is made of or coated with the composite material, has antithrombotic characteristics. By mixing the CNTs, strength and flexibility of the medical instrument can be increased, and operationality and handling properties of the medical instrument can be improved. Even if the medical instrument is implanted in a human body for a long time or semiparmenently, it does not badly influence tissues of the implanted part due to biocompatibility.

### BRIEF DESCRIPTION OF THE DRAWINGS

Fig. 1 is an explanation view of a single layer medical tube.
Fig. 2 is an explanation view of a two-layered medical tube.
Figs. 3A and B are sectional photographs of a control catheter. Figs. 3C and D are sectional photographs of a CNT-nylon composite material.
Fig. 4A is a sectional photograph of an arteria, in which the control catheter is inserted. Fig. 4B is a sectional photograph of an arteria, in which the CNT-nylon composite material is inserted.
Fig. 5A is a sectional photograph showing blood clot around the control catheter. Fig. 5B is a sectional photograph showing blood clot around a new catheter.
Fig. 6A is an SEM photograph showing blood clot around the control catheter. Fig. 5B is an SEM photograph showing blood clot around the new catheter.
Fig. 7A is an SEM photograph of an inner face of the control catheter. Fig. 7B is an SEM photograph of an inner face of the new catheter.
Fig.8 is graphs showing variations of CD4⁺, CD8⁺, and CD4⁺/CD8⁺. Asterisks indicate that the new catheter statistically has significant differences with respect to the control catheter when risk rate is 5 % or less.
Fig. 9 is visual observation views of skin tissues, in which micro catheters are implanted (an upper view: the control catheter; a lower view: the new catheter).
Fig. 10 is left views: optical micrographs of the skin tissue in which the control catheter is implanted, wherein magnifications are different; Right views: optical micrographs of the skin tissue in which the new catheter is implanted, wherein magnifications are different.

### PREFERRED EMBODIMENTS OF THE INVENTION

Preferred embodiments of the present invention will now be described.

Firstly, a composite material of the medical instrument of the present invention will be explained.

The composite material is produced by blending carbon nanotubes with matrix resin and has antithrombotic characteristics in such environments as the medical instrument is brought into contact with blood. When the composite material is implanted in a living body, rejective responsiveness to foreign matters is low and the composit material has excellent biocompatibility. Therefore, the composite material can be used as a suitable antithrombotic material of a medical instrument implanted in a living body.

For example, the matrix resins are: polyester, e.g., polyethylene terephthalate, polybutylene terephthalate; polyester elastomer, in which the polyester constitute hard segments; polyolefin, e.g., polyethylene, polypropylen, and polyolefine elastmer; copolymer polyolefine produced with a metallocene catalyst; vinyl polymer, e.g., polyvinyl chloride, PVDC, PVDF; polyamide and polyamide elastomer (PAE) including nylon; polyimide; polystyrene; SEBS resin; polyurethane; polyurethane elastomer; ABS resin; acrylic resin; polyacrylate; polycarbonate; polyoxymethylene (POM); polyvinyl alcohol (PVA); fluorocarbon resin (ETFE, PFA and PTFE); ethylene binyl acetate; ethylene copolymer vinyl alcohol; ethylene vinyl acetate; carboxymethyl cellulose; methylcellulose; cellulose acetate; vinyl polysulfone; liquid crystal polymer (LCP); polyethersulfone (PES); polyether ether ketone (PEEK); polyphenylene oxide (PPO); thermoplastic resin, e.g., polyphenylene sulfide (PPS), and polymer derivatives thereof; vulcanized rubber; silicon resin; epoxy resin; thermosetting resin, e.g., two-component polyurethane resin; and crosslinking resin.

The nylon may be nylon 6, nylon 64, nylon 66, nylon 610, nylon 612, nylon 46, nylon 9, nylon 11, nylon 12, etc.. Especially, nylon 12 is suitable.

Preferably, the carbon nanotubes (CNT) used in the present invention are produced by a vapor growth method, in which a hydrogen carbonate gas is vapor-thermal-decomposed in the presence of a metallic catalyst.

For example, an organic compound, e.g., benzene, toluene, is used as a raw material, an organic transition metal compound, e.g., ferrocene, nickelocene, is used as the metallic catalyst, and they are introduced into a high-temperature reactor together with a carrier gas so as to produce CNTs on a substrate (see Japanese Patent Kokai Gazette No. 60-27700); CNTs are produced in a floating state (see Japanese Patent Kokai Gazette No. 60-54998); and CNTs are grown on a wall face of a reactor (see Japanese Patent No. 2778434). Further, as disclosed in Japanese Patent Kokoku Gazette No. 3-64606, particles including a metal, which are supported by fire-resistance supporters, e.g., alumina, carbon, may be brought into contact with a carbon compound at high temperature so as to produce CNTs whose diameters are 70 nm or less.

Sizes (diameters) of the CNTs to be used are 1-350 nm, preferably 10-300 nm or 50-200 nm, more preferably 70-160 nm.

If the diameters of the CNTs are smaller than 1 nm, dispersibility in the matrix resin is undesirably lowered. On the other hand, if the diameters thereof are greater than 350 nm, they project from a surface of the matrix resin and asperities are formed therein, so that flatness of the material must be lowered and thin materials (tube-shaped, sheet-shaped, etc.) cannot be formed.

Further, proper aspect ratios of the CNTs are about 50-200.

The CNTs produced by the vapor growth method is graphitized at high temperature. Temperature of the high-temperature treatment is 2000°C or more, preferably 2500 °C or more, more preferably 2800-3200 °C. If the CNTs are treated at temperature of less than 2000 °C, impurities, e.g., amorphous-like deposits, residual metallic catalyst, stick onto surfaces of the CNTs during the vapor-growing process, so they are improper to be used as a material of a medical instrument. By performing the high-temperature treatment at temperature of 2000 °C or more, the impurities, e.g., amorphous-like deposits, residual metallic catalyst, are removed from the surfaces of the CNTs, so they can be used as a proper material of a medical instrument. By treating at temperature of 2500 °C or more, frameworks of the CNTs are graphitized (crystallized), electric conductivity and heat conductivity thereof are improved, so that they can be suitable for many uses, in which electric conductivity and heat conductivity are required.

By graphitizing, flexibility (ductility) can be gained. In case that a tube-shaped medical instrument (e.g., catheter, tube for distributing and transporting blood for blood examination) is made of a material in which the CNTs are blended with matrix resin, the medical instrument, of course, has enough strength, easily restores even if the tube is folded, and it is easily operable (handled) due to the flexible CNTs.

On the other hand, if the high-temperature treatment is performed at temperature of more than 3200 °C, graphite is sublimed, so that graphite frameworks are broken and the strength is undesirably lowered.

Content of iron (Fe) in the CNTs is 500 ppm or less, preferably 150 ppm or less, more preferably 100 ppm or less. If the content is more than 500 ppm, human bodies will be badly influenced, so they cannot be used as a material of medical instruments.

Compounding ratio of the CNTs with respect to the matrix resin is 1-30 wt%, preferably 5-20 wt%, more preferably 7-15 wt%.

If the content of the CNTs is less than 1 wt%, good antithrombotic characteristics cannot be gained. On the other hand, if the content of the CNTs is more than 30 wt%, they cannot be well mixed with the matrix resin, the composite material breaks up and cannot be shaped with insufficient strength. Further, the CNTs are expensive, so a production cost must be undesirably increased.

To uniformly disperse the CNTs in the matrix resin, known means, e.g., a screw type kneader, may be used. To improve dispersibility, ultrasonic vibration may be applied to the mixture of the CNTs and the matrix resin for a prescribed time. Further, to improve adhesiveness of the both, surface structures of the CNTs may be modified by, for example, a shirane coupling agent.

In the composite material, the carbon nanotubes (CNT) form cores for crystallizing the matrix resin and accelerate the crystallization thereof by blending the CNTs with the matrix resin, so that the surface of the matrix resin is made dense and smooth, reactivity with blood is lowered, and excellent antithrombotic characteristics can be gained. When the composite material is implanted in a living body, rejective responsiveness to foreign matters is low, so the composite material has excellent biocompatibility.

The medical instrument of the present invention is characterized in that a section of the medical instrument, a part of or the whole of which is brought into contact with blood, is made of or coated with a composite material, which has antithrombotic characteristics and in which carbon nanotubes are blended with matrix resin.

Namely, as described above, the section of the medical instrument, a part of or the whole of which is brought into contact with blood, is made of the composite material.

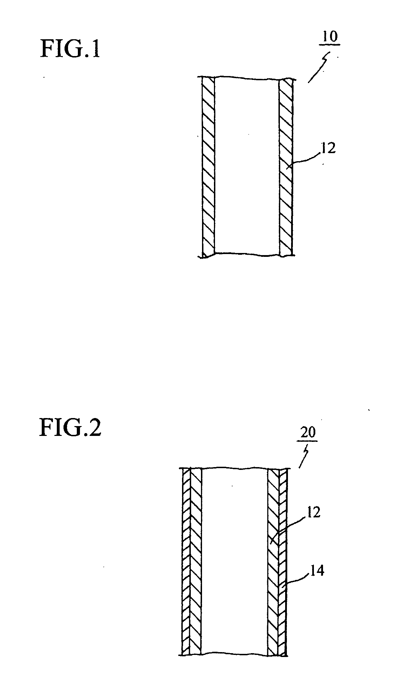

Fig. 1 is a sectional view of a medical tube 10, which is an example of the medical instruments, wherein the whole of the tube 10 is made of the composite material. The tube 10 may be formed by an extrusion molding method.

Fig. 2 is a sectional view of a two-layered medical tube 20, which is an example of the medical instruments, wherein an inner layer 12, which is brought into contact with blood, is made of the composite material, and an outer layer 14 is made of another material, e.g., nylon resin, including no CNTs. Note that, if the outer layer 14 is brought into contact with blood, the outer layer 14 is, of course, made of the composite material. The two-layered tube 20 too can be easily formed by the extrusion molding method.

The medical instruments and parts thereof can be formed by not only the extrusion molding method but also other ordinary methods, e.g., an injection molding method, with the composite material.

In another case, a medical instrument (not shown) may be produced by the step of: forming a base member made of other material by, for example, an injection molding method; and applying the composite material on the base member so as to form the composite material layer, which is brought into contact with blood. In specific medical instruments, e.g., medical stent, pump, pacemaker, which cannot be made of the resin including CNTs due to strength and structures, the sections, which are brought into contact with blood or a human body, can be coated with the composite material.

The coating process is performed by the steps of: preparing the coating material by mixing CNTs with a resin component and a solvent if required; soaking a medical instrument, e.g., stent, in the coating material or applying the coating material thereto; and drying the coating material. For example, thermoplastic resins, e.g., polyethylene, polypropylene, polyamide, polyacrylate, polyvinyl chloride, polyacrylonitrile, polycarbonate, fluorocarbon resin, butadiene resin, may be used as the resin component. Further, at least one of thermosetting resins, e.g., phenol resin, unsaturated polyester resin, epoxy resin, may be added. At least one of solvents selected from a group consisting of: alcohol, e.g., methanol, ethanol; ester solvent, e.g., methyl acetate, ethyl acetate, amyl acetate; ketone solvent, e.g., acetone, methyl acetone; ether solvent, e.g., methyl ether, glycol ethyl ether; hydrocarbon solvent, e.g., kerosene, benzol, xylol, may be used as said solvent. The mixing process is performed by a known mixing means, e.g., mixer, kneader, mill. An order of mixing the members is not limited.

The medical instruments of the present invention will be explained.

Namely, the composite material can be applied to many medical instruments, which are brought into contact with blood, e.g., surgical sheets, surgical knives, pipettes, bed sheets for medical use or menses, cloth, needles for blood drawing, bottles for storing blood, tubes for transporting blood, equipments for blood examinations, catheters (catheter for intravenous hyperalimentation, intravascular catheter, vasodilator catheter, contrast catheter, etc.), tubes of fiber scopes, introducer sheaths, tubes for hemodiafiltration, heart-lung bypass tubes, tubes for distributing and transporting blood, left-ventricular assist devices, vasodilator devices.

Especially, the composite material has the biocompatibility, so it can be effectively used for not only artificial internal organs (e.g., artificial blood vessel, artificial joint) but also medical instruments set (implanted) in human bodies for a long time (e.g., pacemaker, micro pump, drug transporting tube). In a stent, a pump, a pacemaker, etc., which cannot be made of the conventional resin due to strength and structures as described above, their sections which are brought into contact with blood can be coated with the composite material including CNTs; the composite material can be applied to medical instruments for wide medical uses. Since CNTs have high electric conductivity and heat conductivity, the composite material can be applied to other medical instruments.

The medical instruments may have conventional structures, so explanation of their detailed structures will be omitted. Note that, the word "instrument" used in the present invention includes the above described medical devices, their parts, medical tools, ingredients and other medical substances.

### (Examples)

### A. Antithrombotic Test

### 1. CNTs were produced as follows.

A spray nozzle was attached to a top part of a vertical type furnace (inner diameter: 17.0 cm, length: 150 cm). Temperature of an inner wall of the furnace was increased to and maintained at 1200 °C, 20 g/min. of a liquid benzene, which included 4 wt% of ferrocene, was directly supplied (sprayed), with 100 1/min. of a hydrogen gas, toward the inner face of the furnace. At that time, a shape of spray was like a side view of a cone (like a bugle or an umbrella), and an apex angle of the nozzle was 60 degrees. Under said conditions, ferrocene was thermally decomposed to produce iron fine particles (seeds), and carbon fibers were grown from the seeds by carbon produced by thermally decomposing ferrocene. The carbon fibers, which were produced by the vapor growth method, were continuously produced for an hour with whittling every five minutes.

The produced carbon fibers were heat-treated in an argon atmosphere, at temperature of 2800 °C, for 30 minutes. By the heat treatment, high-purity multi-wall CNTs (MWNTs), whose diameters were about 80 nm, lengths were 10-20 µm, specific surface areas (BET method) were 18 m²/g and true specific gravities were 2.08 g/cm³ , were produced. They had relatively linear and long tubular shapes. An amount of metallic impurities (measured by atomic absorption method) was 100 ppm or less (iron was 30 ppm or less).

### 2. Thrombus Formation Test

10 wt% of the carbon nanotubes (CNTs) produced by the above described method were blended with nylon 12, which was the matrix resin. They were blended or mixed by an ordinary mixer. Next, the mixture was extruded and formed into a tubular shape, by a two-shaft extrusion molding machine, so as to form a microcatheter (new catheter), whose inner diameter was 0.46 mm and outer diameter was 0.53 mm.

The antithrobotic formation test was performed in and ex vivo environments with the new catheter and a catheter made of nylon only.

Beagle dogs (adult dogs), whose weights were 9-11 Kg, were used for the test. An anesthetic was injected into veins of the dogs, tubes were inserted into tracheas thereof so as to ventilate, with room air, by a respirator. pH values and O₂-partial pressure of arterial blood were kept within a physiological range with a ventilation speed of (12-15) times/min. and airflow volume of (15-20) ml/kg. Bodily temperatures were maintained at 36°C by heat lamps.

Ordinary vinyl tubes were set in arterias subclavia via arterias brachialis and connected to pressure converters so as to methodically measure blood pressure. Electrocardiograms of Lead II were monitored by bio amplifiers.

Vinyl tubes were set in venas subclavia via venas brachialis so as to inject a lactate Ringer solution into the veins if required. About 5 cm of both faces of thigh bones and ordinary arterias carotis were surgically exposed. The arterias were taped so as to prevent bleeding while they were replaced with the test catheters. The test catheters were inserted into the arterias from center parts toward end parts with angles of about 45 degrees. Therefore, front ends of the catheters were located at centers of lumens of the arterias. Terminal ends of the test catheters were located in outer membranes of blood vessels by the suture so as to prevent bleeding caused by loosening the tapes. The arterias were taken out, together with the test catheters, after a laps of an hour from restarting blood flow. The arterias taken out were fixed in 70 wt% of alcohol and embedded with paraffin. Thin sliced pieces of the samples were dyed with hematoxylin and eosin.

To evaluate blood clotting in a test tube, which was an example of in vitro environment, a new test catheter of 0.8cm was fixed by putting a thin surgical thread through an inner hole of the catheter. 5 ml of venous blood of the dog including no anticoagulant was sampled. Immediately after sampling the blood, the sampled blood was put into a plate, and then the tube was soaked in the blood. Evaluating blood coagulum on the catheter was performed by tenderly lifting the thread upward. To observe by an SEM, the tube was soaked in 2 % of cold a glutaric aldehyde solution in the presence of phosphate buffered saline. Next, the samples were dehydrated by graded ethanol and dried with CO₂ by a critical point method. The dried surfaces were mounted and sputter-coated with gold, palladium and carbon. The samples were sputter-coated with gold and palladium, whose total thicknesses were 50 nm, and observed by a JEOL-JSM 6000 scanning electron microscope at 15 kev.

### 3. Results and Prospect

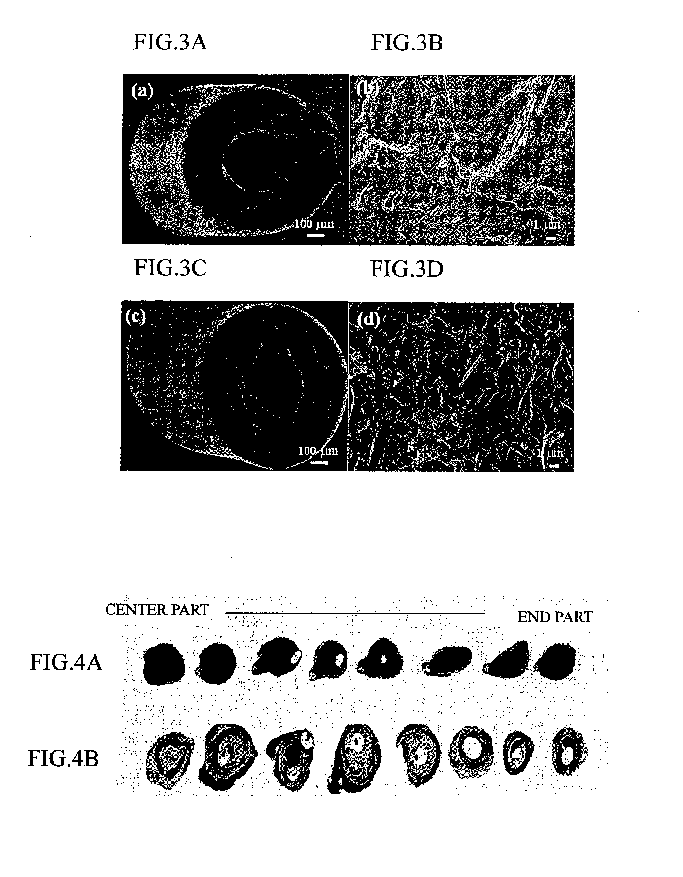

Figs. 3C and D show sections of the catheter (new catheter) made of the nylon-CNT (MWNT) composite material. Relatively uniform dispersion of the CNTs was observed by the FE-SEM (see Fig. 3D). No CNTs were found on the inner and outer walls and projected therefrom. Note that, Figs. 3A and B show sections of a control catheter made of nylon only.

An elastic coefficient of the CNT-based new catheter was about 1.5 times as large as that of the nylon-based control catheter (1200 MPa : 820 MPa). Namely, the CNT-based new catheter has excellent strength, and even if it is once folded, it can rapidly restore, so its operationality (handling property) can be improved.

Fig. 4 shows sections of arterias taken out. In Fig. 4A, the control catheter was in the arteria; in Fig. 4B, the new catheter was in the arteria. In a series of the sections on the upper side (Fig. 4A), deep color parts in the inner space of the arteria indicate prominent thrombus. Places, where the control catheter was inserted, are the sections 3-5 (the section at the far left is section 1), which are shown as opaque holes, and they indicate that the catheter inserted was skewed from the outer membrane to the center of the arteria.

In the control catheter, we think that the thrombus formation was accelerated by rheological variations of blood flow (e.g., turbulence of laminar flow, swirling flow, irregular motion).

On the other hand, in the new catheter, as shown in the sections on the lower side (Fig. 4B), thorombus exists in the section 3 (the third section from the left, in which the catheter was inserted) only. According to the observation, it is obvious that the new catheter weakly reacts to blood if blood flow is not disturbed. Namely, the new catheter is capable of highly restraining thrombus formation in arteria of a living body.

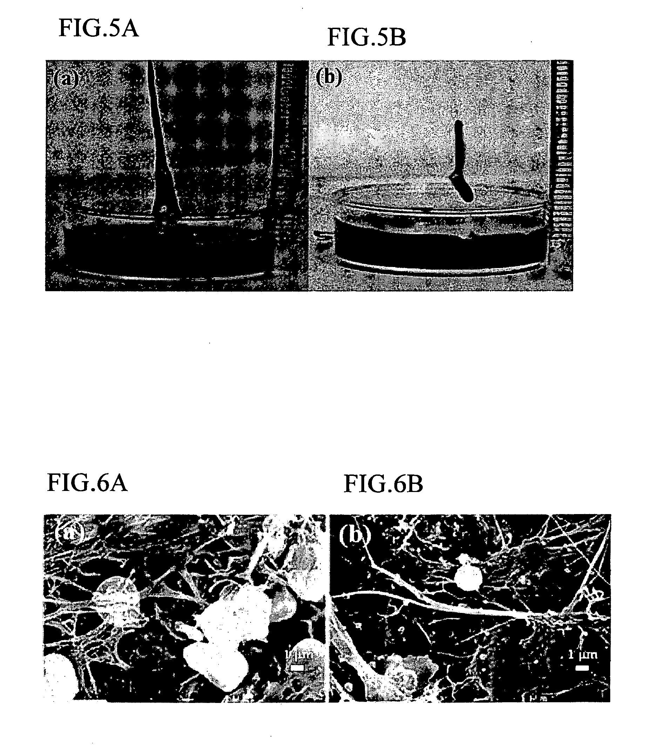

Blood coagulum formation was evaluated by lifting the catheters from the plate. As shown in Fig. 5A, remarkable blood clotting was observed around the control catheter. However, as shown in Fig. 5B, blood reaction of the new catheter was extremely low. To precisely observe blood clotting, the both samples were observed by the SEM (see Fig. 6). Note that, in Fig. 5A, the control catheter was used; in Fig. 5B, the new catheter was used. In the both cases, fibrillose networks and blood cells were observed, but sizes of fibrns and blood cells of the new catheter were relatively small.

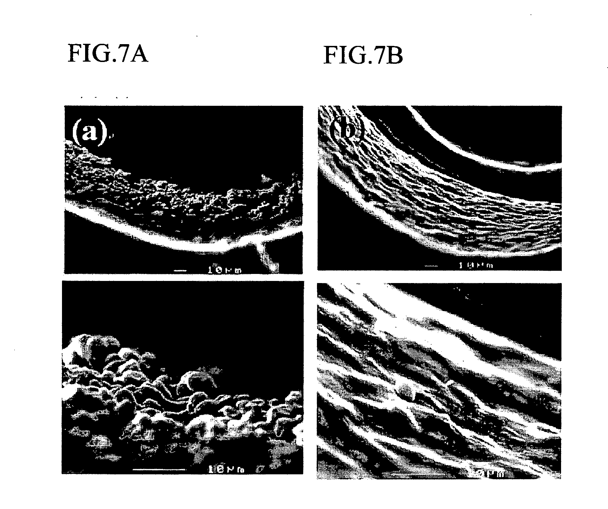

By observing the inner faces of the catheters with the SEM, the inner face of the new catheter was smoother than that of the control catheter (see Fig. 7). Fig. 7A is the control catheter; Fig. 7B is the new catheter. According to the experimental results, we found the following effect. Namely, the catheter, in which CNTs are used as functional fillers, is capable of restraining blood reactions, e.g., blood clotting, fibrin deposition.

### 4. Conclusion

The above described experiments indicate that the new catheter made of the CNT-nylon 12 composite material is capable of improving mechanical properties of the new catheter and restraining blood clotting, etc., and it is highly promising instrument.

CNTs originally have high mechanical strength, so breaking strength of the new catheter can be improved, further the new catheter can easily restore even if it is folded, so that operationality can be improved.

Since the new catheter has the smooth inner face and none of the CNTs project from the outer face thereof, the CNTs are even less likely to be able to directly react with blood. We think that the CNTs form cores for crystallizing the matrix resin (nylon), so that the dense and smooth surfaces can be formed and thrombus formation can be restrained. Further, the improved electrostatic characteristics of the CNTs also restrain thrombus formation. Namely, we think that surface electrical-charge relates to thrombus formation, and electric conductivity of the new catheter is higher than that of the control catheter; the new catheter has no charging property and is hard to be surface-electrical-charged, so that it can have excellent antithrombotic characteristics.

### B. Biocompatibility

Biocompatibility of the new catheter was further studied.

### 1. Experimental Animal, Implanting Method and Samples

We bought 10 female BALB/c mice (five weeks old) from SLC Company (Japan). The mice were kept in a sterile facility of Shinshu University. The experiments were started when the mice were six weeks old. They were divided into two groups: a control catheter group; and a new catheter group. After a lapse of one week from implanting the catheters, samples of blood from hearts and skin tissues were collected from the animals. Back skin of each animal was cut and opened 1 cm with administering a gas-oxygen-fluothane anesthetic. All of the catheters were sterilized by exposing a formaldehyde gas, at room temperature, for 24 hours or more. The catheters were cut to have prescribed sizes (length: about 0.5 mm, weight of the control catheter: 2.3 mg, weight of the new catheter: 1.92 mg), and they were implanted in subcutaneous tissues. The opened wounds were sutured. After a lapse of one week from implanting the catheter in the tissues, the animals were deeply administered with a gas-oxygen-fluothane anesthetic and dissected. To perform flow cytometry measurement, needles were pushed into the hearts so as to collect blood samples, and skin tissues including muscle layers were cut off for pathologic tests.

### 2. Tests

### 2-1. Flow Cytometry

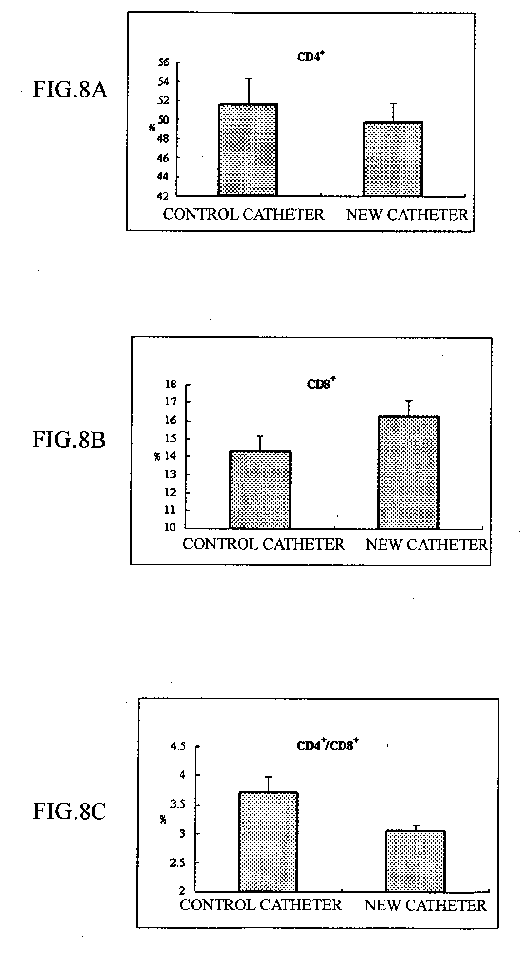

The blood samples were dyed with immune bodies of CD4⁺ and CD8⁺ (produced by BD Bioscience Phermingen Company) so as to sort T-lymph cells by a flow cytometer (produced by FACS Caliver Becton Dickinson and Company, USA). The dyed cells were analyzed by Cell Quest software (produced by Becton Dickinson and Company). Data were indicated as "average value + SE". The differences between the both were evaluated by a statistical technique (risk rate: 5 % or less).
(1) CD4⁺ of Control Catheter Group After 1 Week The values of the T-cells of the control catheter group were 51.5 ± 2.7 %; those of the new catheter group were similar level (i.e., 49.7 ± 2.0 %) (see Fig. 8A).
(2) CD8+
   The values of the control catheter group (after a lapse of one week from the implantation) were 14.3 ± 0.8 %; those of the new catheter group were 16.3 ± 0.9 %, which were significantly greater (see Fig. 8B).
(3) Further, in the control catheter group, ratios (calculated values) between CD4⁺ and CD8⁺ were 3.72 ± 0.25; in the new catheter group, those were 3.07 ± 0.1, which were significantly smaller (see Fig. 8C).
   According to the above described items (1)-(3), MHC Class 1 routes of antigen-antibody reaction path way were enhanced. Namely, reactivity of the new catheter to foreign-body rejection was smaller than that of the control catheter.

Note that, a CD4 is a marker existing on a surface of a helper T-lymph cell, which controls all aspects of immune functions, and CD4 lymph cells accelerate to produce antibodies against antigens. On the other hand, a CD8 is a marker existing on a surface of a suppresser T-lymph cell, and CD8 lymph cells work as brakes so as to limit overproducing antibodies. The ratio between CD4 and CD8 (CD4/CD8) is important for evaluating immune functions; the ratio is lowered when immune functions are compromised. Namely, foreign-body reaction is weakened.

### 2-2. Tissue Structures

Tissues were collected immediately after the dissection as convenience samples of tissue structures, and they were fixed in 10 wt% of formaldehyde and embedded with paraffin, the embeded samples were sliced and formed into cut pieces of five micron, and the cut pieces were dyed with hematoxylin and eosin.

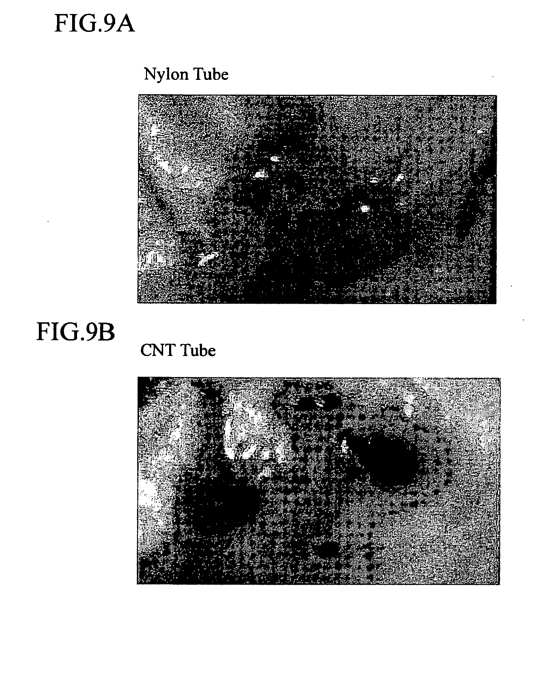

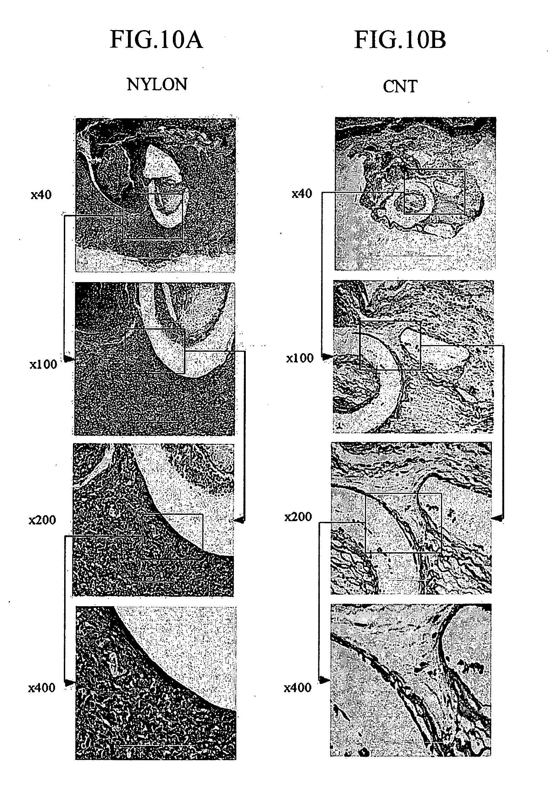

Visual views of the implanted tissues are shown in Fig. 9. In case of the control catheter shown in Fig. 9A, the implanted piece was enclosed by new vascular channels and irritated thick membranes. However, in case of the new catheter shown in Fig. 9B, that phenomenon does not occur. Histological and pathological external views of the implanted microcatheters after a lapse of one week are shown in Fig. 10. Granulation tissues with cellular infiltration remarkably appeared in the control catheter group (see Fig. 10A). In comparison with the new catheter group, the granulation tissues enclosing the control catheter piece were remarkably formed (see Fig. 10B).

### 3. Conclusion

As described above, we confirmed that the new catheter made of the CNT-nylon composite material has the excellent biological characteristics including antithrombotic characteristics and biocompatibility.

## Claims

1. A medical instrument having a section, a part of or the whole of which is brought into contact with blood,
wherein said section is made of or coated with a composite material, which has antithrombotic characteristics and in which carbon nanotubes are blended with matrix resin.

2. The medical instrument according to claim 1,
wherein 1-30 wt% of carbon nanotubes are blended with the matrix resin in the composite material.

3. The medical instrument according to claim 1 or 2,
wherein the carbon nanotubes are graphitized.

4. The medical instrument according to one of claims 1-3,
wherein the carbon nanotubes are produced by a vapor growth method, in which a hydrogen carbonate gas is vapor-thermal-decomposed in the presence of a ferrous catalyst.

5. The medical instrument according to claim 4,
wherein content of iron in the carbon nanotubes is 500 ppm or less.

6. The medical instrument according to one of claims 1-5,
wherein said medical instrument is an implantable instrument, such as an artificial heart, a tube for tranporting a drug and a stent.

7. The medical instrument according to one of claims 1-5,
wherein said medical instrument is an external instrument used outside of a living body, such as an instrument for blood examination and a surgical instrument.
